**Europäisches Patentamt**

**European Patent Office**

**Office européen des brevets**

(19)

(11) Veröffentlichungsnummer: **0 039 916**
**A1**

(12) **EUROPÄISCHE PATENTANMELDUNG**

(21) Anmeldenummer: 81103494.1

(22) Anmeldetag: 07.05.81

(51) Int. Cl.³: **G 01 T 1/164**
**A 61 B 6/02**

(30) Priorität: 09.05.80 US 148261

(43) Veröffentlichungstag der Anmeldung:
**18.11.81 Patentblatt 81/46**

(84) Benannte Vertragsstaaten:
**DE FR GB**

(71) Anmelder: **SIEMENS AKTIENGESELLSCHAFT Berlin und München**
**Postfach 22 02 61**
**D-8000 München 22(DE)**

(72) Erfinder: **Shaw, Howard R.**
**2255 Tasso Street**
**Palo Alto California 94301(US)**

(72) Erfinder: **Morehouse, Charles C.**
**10359 Bonny Drive**
**Cupertino California 95014(US)**

80 P 8914

(54) **Detektorreihe für die Strahlungserfassung.**

(57) Die Strahlendetektorreihe ist modular aufgebaut. Jedes Modul enthält zwei lösbar zusammengebaute Teile (70, 72), von denen einer (72) eine Vielzahl von im Abstand voneinander angeordneten Platten (82) für die Strahlungskollimierung aufweist. Der zweite Teil enthält eine gedruckte Leiterplatte, ein Chip mit einer Halbleiterdiodenreihe, das auf der gedruckten Leiterplatte angeordnet ist, und eine Vielzahl von Szintillationskristallen (84), die auf dem Halbleiterchip angeordnet sind, wobei jeder Kristall (84) auf einer Diode liegt. Die Signale von den Dioden werden Signalverarbeitungsmitteln mit Hilfe eines Kabels (95) zugeführt, das in einfacher Weise mit der Diodenreihe verbunden oder von dieser gelöst werden kann. Es ist eine verbesserte räumliche Auflösung erreicht und der Zusammenbau und der Ersatz für die Reparatur erleichtert.

Croydon Printing Company Ltd

SIEMENS AKTIENGESELLSCHAFT          Unser Zeichen
Berlin und München                  VPA 80 P 8914 E

Detektorreihe für die Strahlungserfassung

Die Erfindung bezieht sich auf eine Detektorreihe für
die Strahlungserfassung in einem Strahlendiagnostikgerät.

In Computertomographen wird eine Röntgenstrahlen-Abtastvorrichtung benutzt, die ein divergentes, fächerförmiges Röntgenstrahlenbündel durch einen Patienten
richtet, wobei die aus dem Patienten austretende Strahlung von einem Strahlendetektor erfaßt wird, der der
Strahlenquelle gegenüberliegend angeordnet ist. Die
Strahlenquelle und der Detektor rotieren um den Patienten und Projektionsdaten werden bei einer Vielzahl von
Winkelstellungen erhalten. Die Projektionsdaten werden
dann von einem Computer verarbeitet, um ein Bild der
durchstrahlten Schicht des Patienten zu erhalten.

In den US-Patentschriften 40 75 491 und 41 90 772 sind
solche tomographische Abtastgeräte beschrieben, bei
denen die Strahlenquelle und der Strahlendetektor drehbar gelagert sind, um einen Patienten in einer Vielzahl von Winkelstellungen abzutasten. Um mehrere Abtastungen für eine feste Position der Strahlenquelle
zu erhalten, sind Verschiebungsmittel benutzt, um die
Position des Detektors auf dem tomographischen Gerät
einzustellen. In beiden Patenten ist der Detektor eine
einheitliche Struktur, wie eine mit Xenon gefüllte Kammer mit einer Vielzahl von Anoden und Kathoden, die
Detektorelemente definieren. Die Verwendung von Szintillationskristallen mit Fotomultipliern oder Foto-

Tp 5 Ler / 04.05.1981

dioden für die Detektorelemente in der einheitlichen Struktur wird in dem Patent 41 90 772 vorgeschlagen.

Die Strahlendetektoren, die hierfür vorgeschlagen sind, haben verschiedene Nachteile. Die einheitlichen Strukturen sind während des Zusammenbaues und während der Verschiebung der Detektorposition schwer zu handhaben, um eine Mehrzahl von Positionsabtastungen zu erzielen. Der Modifikation der Detektorreihe sind dadurch Grenzen gesetzt, daß eine einheitliche Struktur vorgesehen ist. Ferner erfordert der Ausfall irgendeines Detektorelementes den Ersatz des gesamten Detektors und die Reparatur des defekten Elementes wird durch die einheitliche Struktur erschwert.

Demgemäß ist es Aufgabe der vorliegenden Erfindung, eine Strahlendetektorreihe so zu verbessern, daß sie leicht zu reparieren ist.

Entsprechend der vorliegenden Erfindung ist diese Aufgabe dadurch gelöst, daß sie eine Vielzahl von Detektormodulen aufweist, von denen jedes eine Vielzahl von Fotodioden und eine Vielzahl von Szintillationskristallen enthält, wobei jeder Szintillationskristall mit einer der Fotodioden zusammenarbeitet und die von einem Szintillationskristall empfangene Strahlung Fotonen in ihm erzeugt, die zu einer der Fotodioden übertragen werden. Die erfindungsgemäße Detektorreihe enthält eine Vielzahl von Detektormodulen, wobei ein defektes Modul leicht ersetzt werden kann, ohne daß die Operationsfähigkeit der Detektorreihe beeinflußt wird. Aufgrund des modularen Aufbaues der Detektorreihe sind die Installation und die Positionierung der Reihe in einem tomographischen Gerät erleichtert.

Jedes Modul der Detektorreihe enthält eine Vielzahl von Fotodioden und eine Vielzahl von Szintillationskristallen, wobei jeder Szintillationskristall mit einer der Fotodioden zusammenarbeitet, und die von einem Szintillationskristall empfangene Strahlung Fotonen erzeugt, die zu einer der Fotodioden übertragen werden. Jedes Modul kann auch eine gedruckte Leiterplatte enthalten, sowie Mittel zur Verbindung der Vielzahl von Fotodioden mit der gedruckten Leiterplatte und einen elektrischen Verbinder, der auf der gedruckten Leiterplatte angeordnet ist und mit dem die Vielzahl von Fotodioden elektrisch verbunden ist. Auf diese Weise kann das Modul leicht in die Detektorreihe eingefügt und aus dieser entfernt werden.

Ein Strahlungskollimator kann mit den Szintillationskristallen zusammenarbeiten, wobei der Kollimator jedem Szintillationskristall kollimierte Strahlung zuführt.

In einer bevorzugten Ausführungsform enthält das Modul einen ersten Teil, in dem die gedruckte Leiterplatte, die Fotodiodenreihe und die Szintillationskristalle angeordnet sind. Ein zweiter Teil enthält dabei den Kollimator und ist lösbar mit dem ersten Teil zusammengebaut, wobei die zwei zusammengebauten Teile in einem tomographischen Gerät in einfacher Weise montiert werden können.

Die Erfindung ist anhand eines in den Zeichnungen dargestellten Ausführungsbeispieles näher beschrieben. Es zeigen:

Fig. 1 eine perspektivische Ansicht eines tomographischen Gerätes, in dem die Erfindung anwendbar ist,

- 4 -        VPA 80 P 8914 E

Fig. 2 eine Ansicht einer Ausführungsform einer
Detektorreihe nach der Erfindung von oben,

Fig. 3 eine Ansicht der Detektorreihe der Figur 2
von vorne,

Fig. 4 eine Seitenansicht der Detektorreihe der

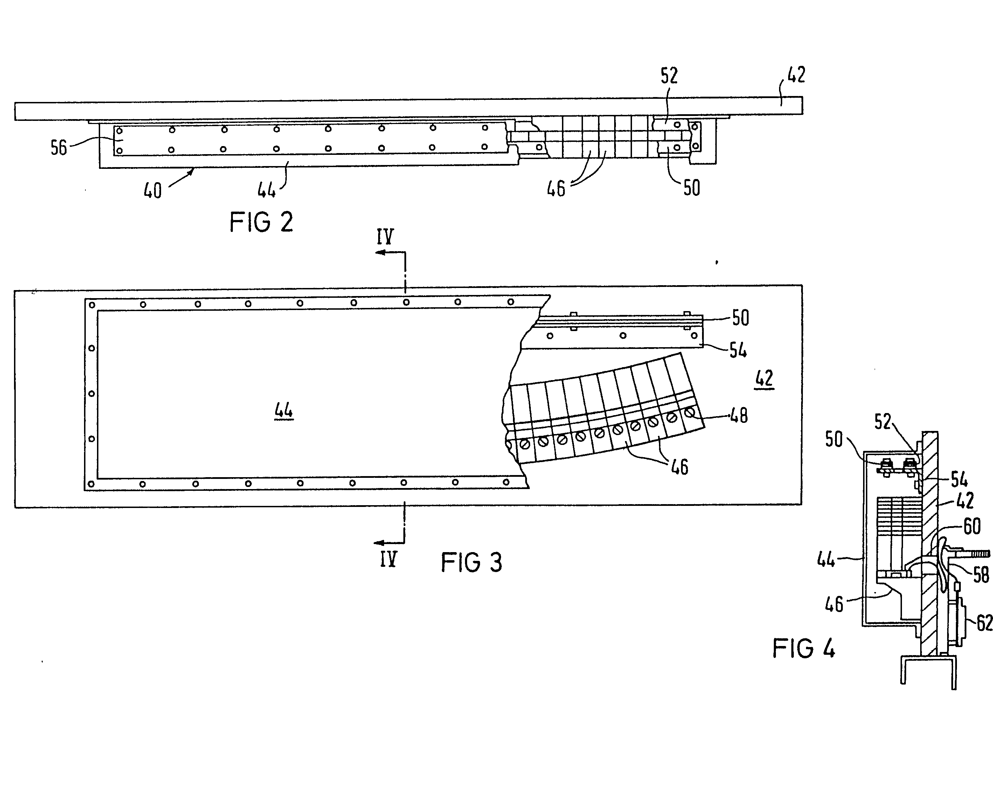

Figuren 2 und 3 im Schnitt,

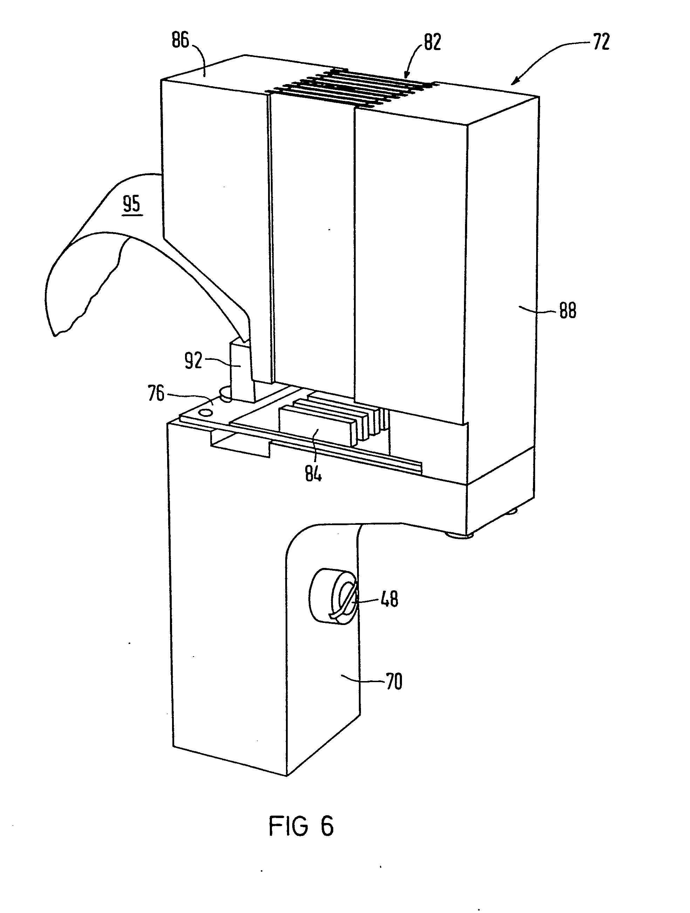

Fig. 5 eine Vorderansicht einer Basisplatte, auf
der die Detektorreihe der Figuren 2 und 4
montiert ist,

Fig. 6 eine perspektivische Ansicht einer Ausführungsform eines Detektormodules der Detektorreihe nach der Erfindung,

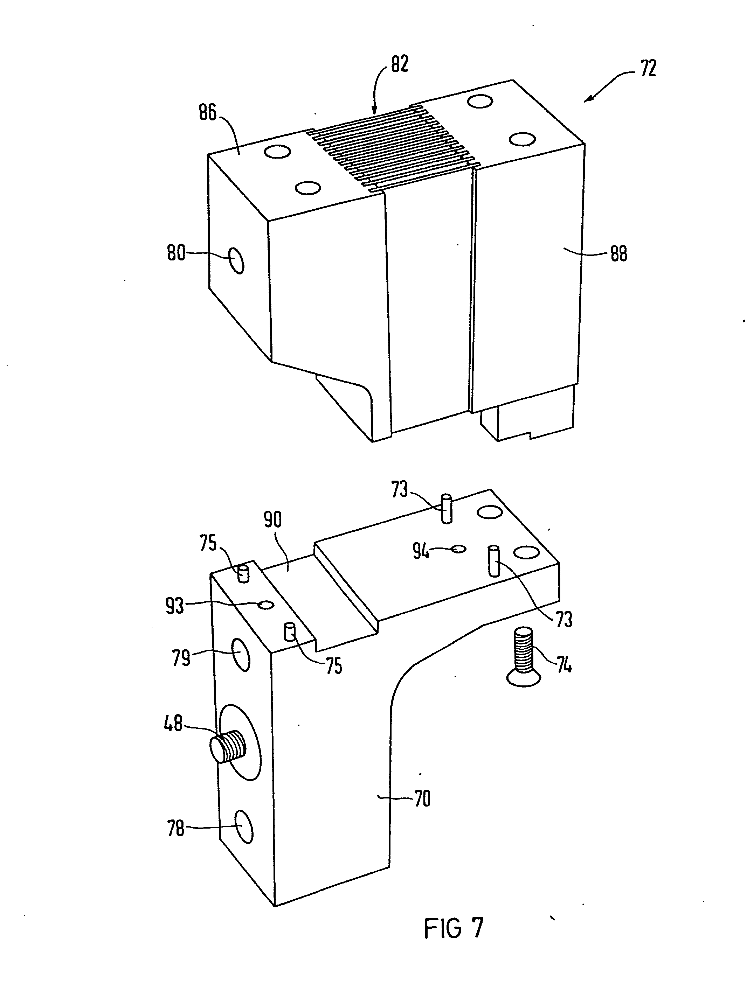

Fig. 7 eine perspektivische Explosionsdarstellung,
die Teile des Detektormodules der Figur 6
zeigt, und

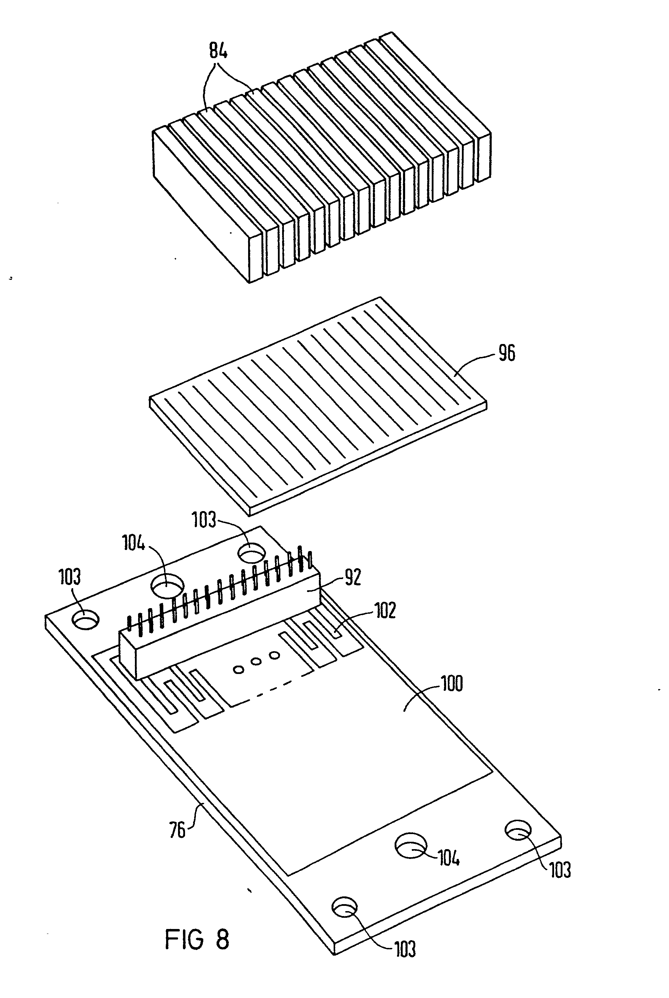

Fig. 8 eine Explosionsdarstellung der Szintillationskristalle, der Fotodioden und der gedruckten Leiterplatte eines Teiles des Modules der Figur 6.

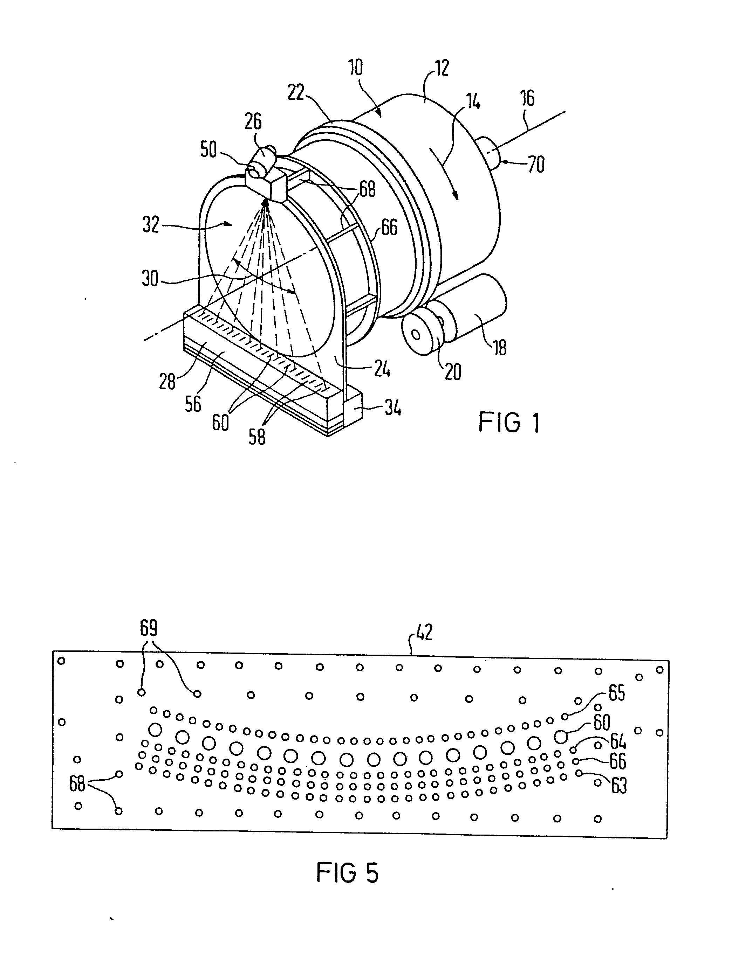

In der Figur 1 ist eine perspektivische Darstellung
eines tomographischen Abtastgerätes gezeigt, wie es im
US-Patent 41 90 772 beschrieben ist. Das Gerät enthält
eine rotierende Einheit 10, die einen äußeren Zylinder
12 aufweist, der in einer Richtung 14 um die zentrale
Achse 16 rotiert und zwar mit Hilfe eines Motors 18
und eines Antriebsrades 20, das mit einem Antriebsring
22 in Eingriff steht, der auf dem Zylinder 12 befestigt
ist. Auf einer Basisplatte 24 sind eine Strahlenquelle 26 und ein Detektor 28 angeordnet, der der Strahlenquelle 26 gegenüberliegt. Die Strahlenquelle 26 richtet ein Röntgenstrahlenbündel 30 in Form eines Strahlenfächers auf den Detektor 28.

Während des Betriebes liegt ein Patient in der zentralen Öffnung 32 zwischen der Strahlenquelle 26 und dem
Detektor 28. Durch Rotation der Strahlenquelle 26 und
des Detektors 28 um den Patienten werden in einer Vielzahl von winkelmäßig versetzten Positionen einem Schattenbild entsprechende Daten erhalten, aus denen ein
Schichtbild der durchstrahlten Schicht des Patienten
durch einen Computer rekonstruiert wird.

Bei bekannten Geräten enthält der Detektor eine einheitliche Reihe aus Ionisationskammern, wie Xenon-
Krypton-Detektoren, die die empfangene Strahlung in
elektrische Signale umwandeln, welche dann einem Speicher in Signalverarbeitungsmitteln 34 zugeführt werden. Der Detektor ist relativ schwer, was den Zusammenbau und die Justierung des Detektors auf der Basisplatte 24 schwierig macht.

Entsprechend der vorliegenden Erfindung ist eine modulare Strahlendetektorreihe vorgesehen, die leichter
installiert und gewartet werden kann. Die Figuren 2, 3
und 4 zeigen in einer Ansicht von oben, einer Vorderansicht und einer Seitenansicht eine Ausführungsform
einer modularen Strahlendetektorreihe, die insgesamt
mit 40 bezeichnet ist und auf einer Basisplatte 42 des
Abtastgerätes befestigt ist. Ein Teil der Abdeckung 44
ist in jeder der Darstellungen entfernt, um die Anordnung der Module 46 der Detektorreihe zu zeigen. Jedes
der Module 46 ist mit der Basisplatte 42 verbunden,
und zwar mit Hilfe einer Schraube 48 in Verbindung mit Justierstiften, die sich von der Basisplatte 42 aus erstrecken, wie dies später in Verbindung mit der Figur 5 näher beschrieben ist.

Über der modularen Detektorreihe 40 ist ein Paar von Strahlungsabschirmungen 50 und 52 angeordnet, das auf der Basisplatte 42 mit Hilfe einer Klammer 54 befestigt ist. Die Strahlungsabschirmungen 50 und 52 können aus Blei mit rostfreien Stahlkanten zur Verstärkung bestehen, wobei der Raum dazwischen ein fächerförmiges Strahlenbündel definiert, das zu der Detektorreihe 40 verläuft. Die obere Oberfläche der Abdeckung 44 enthält eine optische undurchlässige Wand 56, wie Plastikmaterial, die die Übertragung von Röntgenstrahlung erlaubt, aber Licht von der eingeschlossenen Detektorreihe 40 fernhält.

Wie in der Figur 4 dargestellt ist, ist jedes der Module 46 mit einem elektrischen Kabel 58 verbunden, das durch eine Öffnung 60 in der Basisplatte 42 verläuft und zu einen Verbinder 62 geführt ist. Wie noch später beschrieben wird, kann jedes Modul 46 einfach im Abtastgerät eingefügt und aus diesem entfernt werden, so daß der Zusammenbau und die Wartung vereinfacht sind.

In der Figur 5 ist eine Frontansicht der Basisplatte 42 dargestellt, wobei die modulare Detektorreihe 40 entfernt ist, um ihre Verbindung mit der Basisplatte 42 näher zu erläutern. Die Basisplatte 42 weist drei Reihen von Justierstiften 63, 64, 65 auf, wobei ein Stift in jeder Reihe ein Detektormodul 46 trägt und ausrichtet. Die Schraube 48, die jedes Modul 46 mit der Basisplatte 42 verbindet, greift in ein Loch in der Reihe 66 ein. Auf diese Weise wird jedes Modul 46 durch drei Stifte 63, 64, 65 ausgerichtet und von der Schraube 48 getragen, die es mit der Basisplatte 42 verbindet. Die Öffnungen 60 in der Basisplatte 42 erlauben den Durchtritt der Kabel 58 von jedem Modul. Löcher 69 nehmen Schrauben für die Verbindung der Abdeckung 44 mit der Basisplatte 42 auf und Löcher 69 nehmen Schrauben für die Verbindung der Klammer 54 mit der Basisplatte 42 auf.

Die Figuren 6 und 7 zeigen perspektivische Ansichten eines Detektormodules 46 im Zusammenbau und in einer Explosionsdarstellung. Das Modul 46 enthält eine Basis 70 als ersten Teil und einen zweiten Teil 72, der lösbar mit der Basis 70 verbunden ist, und zwar mit Hilfe eines Paares von Justierstiften 73 und Schrauben 74. Die Basis 70 enthält ferner ein zweites Paar von Stiften 75, die mit Stiften 73 zusammenwirken, um eine gedruckte Leiterplatte 76 auszurichten und zu halten, auf der Szintillationskristalle 84 und Fotodioden angeordnet sind. Die gedruckte Leiterplatte 76 ist in der Explosionsdarstellung der Figur 7 entfernt, um die Basis 70 genauer zu zeigen. Die Justierstifte 63, 64, 65 erstrecken sich von der Basisplatte 42, wie dies in der Figur 5 dargestellt ist, und sind in Löchern 78, 79 und 80 geführt, die die Figur 7 zeigt. Die Schraube 48 erstreckt sich durch die Basis 70, um das zusammengebaute Modul mit der Basisplatte 42 zu verbinden.

Der Teil 72 des Detektormodules enthält eine Vielzahl von im Abstand voneinander angeordneten Platten 82, wobei einander benachbarte Platten 82 einen Pfad zum Durchlaß kollimierter Strahlung zu einem Szintillationskristall 84 bilden, das auf der gedruckten Leiterplatte 76 angeordnet ist. Die Platten 82 können aus rostfreiem Stahl bestehen und sind dauerhaft in Nuten einander gegenüberliegender Oberflächen von Traggliedern 86 und 88 mit Hilfe eines Kunstharzes befestigt. Der Raum zwischen den Platten 82 ist auf die Breite eines Szintillationskristalles 84 abgestimmt und die Platten 82 werden in Bezug auf den zugeordneten Szintillationskristall 84 mit Hilfe der Stifte 73 und der Schrauben 74 ausgerichtet, die die zwei zusammengefügten Teile 70 und 72 des Detektormodules 46 in Bezug aufeinander ausrichten und miteinander verbinden. Obwohl ein Kollimator bei dem Ausführungsbeispiel dargestellt ist, ist die Verwendung eines Kollimators in dem Detektormodul 46 nicht wesentlich.

Eine Nut 90 in der oberen Oberfläche der Basis 70 nimmt einen Verbinder 92 auf, der auf der gedruckten Leiterplatte 76 angeordnet ist und Löcher 93 und 94 in der oberen Oberfläche der Basis 70 nehmen Schrauben für die Befestigung der gedruckten Leiterplatte 76 auf der Basis 70 auf. Ein Bandkabel 95 erstreckt sich vom Oberteil des Verbinders 92 aus zu den Signalverarbeitungsmitteln 34 der Figur 1.

Die Figur 8 zeigt eine Explosionsdarstellung der gedruckten Leiterplatte 76, eines Fotodioden-Halbleiterchips 96 und der Szintillationskristalle 84. Die gedruckte Leiterplatte 76 hat einen Teil 100, der ein Metallmuster mit Leitern 102 aufweist. Der Fotodioden-Halbleiterchip 96 wird mit dem Teil 100 mit einem geeigneten leitfähigen Harz verbunden und die Kontakte für jede der Fotodioden des Halbleiterchips 96 sind Drähte, die mit den Leitern 102 verlötet sind, um die Fotodioden mit dem Verbinder 92 zu verbinden. Löcher 103 in der gedruckten Leiterplatte 76 nehmen die Justierstifte 73 und 75 der Basis 70 auf, wie dies in der Figur 7 gezeigt ist, und Löcher 104 nehmen Schrauben auf, die in Löcher 93 und 94 der Basis 70 eingreifen, um die gedruckte Leiterplatte 76 auf der Basis 70 zu befestigen. Jeder Szintillationskristall 84 ist mit dem Halbleiterchip 96 durch geeignete Mittel verbunden, wobei jeder Szintillationskristall 84 auf eine Fotodiode ausgerichtet ist. Jeder Szintillationskristall 84 hat Dimensionen, die geeignet sind, eine Fotodiode voll abzudecken, so daß die Diode Fotonen nur von dem zugeordneten Szintillationskristall 84 empfängt.

Der Halbleiterchip 96 enthält eine integrierte Diodenstruktur, die handelsüblich ist. Der Verbinder 92 ist ebenfalls handelsüblich.

Bei einer Ausführungsform sind sechzehn Kanäle in jedem Modul 46 vorgesehen, so daß das Modul 46 sechzehn Dioden aufweist und die Strahlung innerhalb von einem Grad des fächerförmigen Strahlenbündels empfängt. Weil die beschriebenen Festkörper-Detektorelemente kleiner als Xenon-Detektoren sind, erlaubt die höhere Dichte eine bessere räumliche Auflösung im Hinblick auf konventionelle Detektoren. Die modulare Konstruktion der Detektorreihe erlaubt, daß jedes Modul in einfacher Weise von dem Abtastgerät für Reparatur und Ersatz entfernt werden kann.

8 Figuren
25 Patentansprüche

Patentansprüche

1. Detektorreihe für die Strahlungserfassung in einem Strahlendiagnostikgerät, d a d u r c h  g e k e n n - z e i c h n e t , daß sie eine Vielzahl von Detektormodulen (46) aufweist, von denen jedes eine Vielzahl von Fotodioden (96) und eine Vielzahl von Szintillationskristallen (84) enthält, wobei jeder Szintillationskristall (84) mit einer der Fotodioden (96) zusammenarbeitet und die von einem Szintillationskristall (84) empfangene Strahlung Fotonen in ihm erzeugt, die zu einer der Fotodioden (96) übertragen werden.

2. Detektorreihe nach Anspruch 1, d a d u r c h g e k e n n z e i c h n e t , daß jedes der Module (46) eine gedruckte Leiterplatte (76) aufweist, wobei Mittel vorgesehen sind, die mehrere der Fotodioden (96) mit der gedruckten Leiterplatte (76) verbinden und ein elektrischer Verbinder (92) auf der gedruckten Leiterplatte befestigt ist, an dem die Fotodioden (96) elektrisch angeschlossen sind.

3. Detektorreihe nach Anspruch 1, d a d u r c h g e k e n n z e i c h n e t , daß die Detektormodule (46) mit dem tomographischen Gerät verbunden sind, um ein divergentes Strahlenbündel (30) zu erfassen.

4. Detektorreihe nach Anspruch 3, d a d u r c h g e k e n n z e i c h n e t , daß eine Abdeckung (44) für die in dem tomographischen Gerät angeordneten Detektormodule (46) vorhanden ist, wobei Mittel (50, 52) zur selektiven Übertragung eines fächerförmigen Strahlenbündels (30) zu den Einzeldetektoren vorhanden sind.

5. Detektorreihe nach Anspruch 4, d a d u r c h
g e k e n n z e i c h n e t , daß die Mittel zur
Übertragung eines fächerförmigen Strahlenbündels (30)
ein Paar von im Abstand zueinander angeordneten Strahlungsabschirmungen (50, 52) aufweisen, und das fächerförmige Strahlenbündel (30) von dem Abstand zwischen
den Strahlungsabschirmungen (50, 52) definiert ist.

6. Detektorreihe nach Anspruch 5, d a d u r c h
g e k e n n z e i c h n e t , daß die Abdeckung
(44) eine optisch undurchlässige Wand (56) über dem
Paar von Strahlungsabschirmungen (50, 52) aufweist.

7. Detektorreihe nach Anspruch 1, d a d u r c h
g e k e n n z e i c h n e t , daß eine Vielzahl von
Strahlungskollimatoren (82) vorhanden ist, die in Bezug auf die Szintillationskristalle (84) in der Weise
angeordnet sind, daß jeder Kollimator (82) kollimierte Strahlung zu einem Szintillationskristall (84)
führt.

8. Detektorreihe nach Anspruch 7, d a d u r c h
g e k e n n z e i c h n e t , daß jedes der Detektormodule (46) einen ersten Teil (70) aufweist, auf
dem eine Vielzahl von Fotodioden (96) und eine Vielzahl von Szintillationskristallen (84) auf den Fotodioden (96) angeordnet sind, und wobei ein zweiter
Teil (72) vorgesehen ist, der den Strahlungskollimator (82) enthält und der mit dem ersten Teil (70) lösbar verbunden ist.

9. Detektorreihe nach Anspruch 8, d a d u r c h
g e k e n n z e i c h n e t , daß im zweiten Teil
(72) eine Vielzahl von im Abstand voneinander angeordneten Platten (82) und Tragmittel (86, 88) an ein-

- 12 -     VPA 80 P 8914 E

ander gegenüberliegenden Enden der Platten (82) angeordnet sind, um die Platten (82) im Abstand voneinander zu halten.

10. Detektorreihe nach Anspruch 9, d a d u r c h
g e k e n n z e i c h n e t , daß benachbarte Platten (82) einen Kollimierungspfad definieren, um die
Strahlung (30) auf einen Szintillationskristall (84)
zu richten.

11. Detektorreihe nach Anspruch 8, d a d u r c h
g e k e n n z e i c h n e t , daß der erste Teil (70)
eine gedruckte Leiterplatte (76) enthält und Mittel
zur Verbindung der Vielzahl von Fotodioden (96) mit
der gedruckten Leiterplatte (76) vorhanden sind, wobei ein elektrischer Verbinder (92) auf der gedruckten Leiterplatte (76) angeordnet ist und die Fotodioden (96) mit dem Verbinder (92) elektrisch verbunden sind.

12. Detektorreihe nach Anspruch 11, d a d u r c h
g e k e n n z e i c h n e t , daß die gedruckte
Leiterplatte (76) eine Vielzahl von Justierlöchern
(103) aufweist und der erste Teil (70) mit einer Vielzahl von Stiften (73, 75) versehen ist, die in den
Justierlöchern (103) der gedruckten Leiterplatte (76)
liegen.

13. Detektorreihe nach Anspruch 12, d a d u r c h
g e k e n n z e i c h n e t , daß der zweite Teil
(72) eine Vielzahl von Löchern aufweist, in die die
Stifte (73, 75) des ersten Teiles (70) eingreifen.

14. Detektorreihe nach Anspruch 8 oder 12, d a -
d u r c h  g e k e n n z e i c h n e t , daß Mittel

(42) vorhanden sind, die die Detektormodule (46) in dem tomographischen Gerät halten, um ein divergentes Strahlenbündel (30) zu erfassen.

15. Detektorreihe nach Anspruch 14, d a d u r c h g e k e n n z e i c h n e t , daß eine Abdeckung (44) vorhanden ist, die eine Vielzahl von Detektormodulen (46), die im tomographischen Gerät angeordnet sind, aufnimmt, und bei der Mittel (50, 52) zur selektiven Übertragung eines fächerförmigen Strahlenbündels (30) zu den Einzeldetektoren vorhanden sind.

16. Detektorreihe nach Anspruch 15, d a d u r c h g e k e n n z e i c h n e t , daß die Mittel zur selektiven Übertragung eines fächerförmigen Strahlenbündels (30) ein Paar von im Abstand voneinander angeordneten Abschirmungen (50, 52) aufweisen, wobei das fächerförmige Strahlenbündel (30) durch den Abstand der Strahlungsabschirmungen (50, 52) definiert ist.

17. Detektorreihe nach Anspruch 16, d a d u r c h g e k e n n z e i c h n e t , daß die Abdeckung (44) eine optisch undurchlässige Wand (56) über dem Paar von im Abstand voneinander angeordneten Strahlungsabschirmungen (50, 52) aufweist.

18. Detektormodul für die Anordnung in einer modularen Detektorreihe nach Anspruch 1, d a d u r c h g e k e n n z e i c h n e t , daß es eine Vielzahl von Fotodioden (96) und eine Vielzahl von Szintillationskristallen (84) aufweist, wobei jeder Szintillationskristall (84) mit einer der Fotodioden (96) zusammenarbeitet und die von einem Szintillationskristall (84) empfangene Strahlung Fotonen darin erzeugt,

- 14 -          VPA 80 P 8914 E

die zu einer der Fotodioden (96) übertragen werden.

19. Detektormodul nach Anspruch 18, d a d u r c h
g e k e n n z e i c h n e t , daß ein Strahlungskollimator (82) vorhanden ist, der mit den Szintillationskristallen (84) zusammenwirkt, wobei der Kollimator (82) kollimierte Strahlung zu den Szintillationskristallen führt.

20. Detektormodul nach Anspruch 19, d a d u r c h
g e k e n n z e i c h n e t , daß es einen ersten
(70) und einen zweiten Teil (72) aufweist, wobei der
erste Teil (70) eine Vielzahl von Fotodioden (96) und
eine Vielzahl von Szintillationskristallen (84) auf
den Fotodioden (96) enthält und der zweite Teil (72)
den Strahlungskollimator (82) aufweist und der erste
(70) und der zweite Teil (72) lösbar miteinander verbunden sind.

21. Detektormodul nach Anspruch 20, d a d u r c h
g e k e n n z e i c h n e t , daß der zweite Teil
(72) eine Vielzahl von im Abstand zueinander angeordneten Platten (82) und Tragmittel (86, 88) an einander gegenüberliegenden Enden der Platten (82) aufweist, um die Platten (82) im Abstand voneinander zu
halten.

22. Detektormodul nach Anspruch 21, d a d u r c h
g e k e n n z e i c h n e t , daß der erste Teil
(70) eine gedruckte Leiterplatte (76) aufweist und
Mittel vorhanden sind, die die Vielzahl von Fotodioden
(96) mit der gedruckten Leiterplatte (76) verbinden
und ein elektrischer Verbinder (92) auf der gedruckten Leiterplatte (76) angeordnet ist, mit dem die
Fotodioden (96) elektrisch verbunden sind.

23. Detektormodul nach Anspruch 22, d a d u r c h g e k e n n z e i c h n e t , daß die gedruckte Leiterplatte (76) eine Vielzahl von Justierlöchern (103) und der erste Teil (70) eine Vielzahl von Stiften (73, 75) aufweist und die gedruckte Leiterplatte (76) mit den Stiften (73, 75) in den Löchern (103) hält.

24. Detektormodul nach Anspruch 23, d a d u r c h g e k e n n z e i c h n e t , daß der zweite Teil (72) eine Vielzahl von Löchern aufweist, die mit den Stiften (73, 75) des ersten Teiles (70) in Eingriff stehen.

25. Detektormodul nach Anspruch 18, d a d u r c h g e k e n n z e i c h n e t , daß eine gedruckte Leiterplatte (76) vorhanden ist, mit der die Vielzahl von Fotodioden (96) verbunden ist, wobei ein elektrischer Verbinder (92) auf der gedruckten Leiterplatte (76) angeordnet ist und Mittel vorhanden sind, die die Vielzahl von Fotodioden (96) mit dem Verbinder (92) elektrisch verbinden.

FIG 1

FIG 5

0039916

FIG 2

42

52

50

46

44

40

56

FIG 3

42

50

54

48

46

44

IV

IV

FIG 4

54

42

60

58

62

52

50

44

46

FIG 6

FIG 7

FIG 8

**0039916**

Europäisches
Patentamt

**EUROPÄISCHER RECHERCHENBERICHT**

Nummer der Anmeldung

EP 81 10 3494

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | <u>US - A - 4 181 856</u> (P.R. BONE)<br><br>  * Figuren 4,5 *<br><br>  -- | 1-3,<br>11,18,<br>22,25 |
| | <u>GB - A - 2 030 422</u> (SIEMENS A.G.)<br><br>  * Figuren 1-6 *<br><br>  -- | 1-5,7-<br>11,14-<br>16,18-<br>22,25 |
| | <u>GB - A - 2 005 953</u> (SIEMENS A.G.)<br><br>  * Zusammenfassung; Figur 4 *<br><br>  -- | 3-7,9,<br>10,14-<br>17,19,<br>21 |
| P | <u>FR - A - 2 455 292</u> (THOMSON-CSF)<br><br>  * Seite 5, Zeile 27 - Seite 6,<br>  Zeile 14 *<br><br>  ---- | 1-3,<br>11,18,<br>22,25 |

**KLASSIFIKATION DER ANMELDUNG (Int Cl³)**

G 01 T 1/164
A 61 B 6/02

**RECHERCHIERTE SACHGEBIETE (Int Cl³)**

G 01 T 1/164
A 61 B 6/02

**KATEGORIE DER GENANNTEN DOKUMENTE**

X: von besonderer Bedeutung

A: technologischer Hintergrund

O: nichtschriftliche Offenbarung

P: Zwischenliteratur

T der Erfindung zugrunde liegende Theorien oder Grundsätze

E: kollidierende Anmeldung

D in der Anmeldung angeführtes Dokument

L aus andern Gründen angeführtes Dokument

& Mitglied der gleichen Patentfamilie, übereinstimmendes Dokument

| | Der vorliegende Recherchenbericht wurde für alle Patentansprüche erstellt |
|---|---|

| Recherchenort | Abschlußdatum der Recherche | Prüfer |
|---|---|---|
| Den Haag | 05-08-1981 | ARMSPACH |

EPA form 1503.1  06.78